# EUROPEAN PATENT SPECIFICATION

(11) **EP 3 247 309 B1**
(45) Date of publication and mention of the grant of the patent: **08.03.2023**
(21) Application number: 16740891.3
(22) Date of filing: 23.01.2016
(51) Int. Cl.: A61B 5/00, A61B 5/103, A61B 5/145, A61B 5/20, A61F 2/04, A61F 2/00

(54) **BLADDER MANAGEMENT SYSTEMS**
BLASENMANAGEMENTSYSTEME
SYSTÈMES DE PRISE EN CHARGE DE LA VESSIE

(30) Priority: 23.01.2015 US 201562107203 P; 01.04.2015 US 201562141520 P; 16.07.2015 US 201562231854 P; 06.01.2016 US 201662275671 P; 15.01.2016 US 201662279485 P
(43) Date of publication of application: 29.11.2017
(73) Proprietor: Spinal Singularity, Inc., San Clemente, CA 92672 (US)
(72) Inventor: HERRERA, Derek, San Clemente, CA 92672 (US); SHEN, Alex, Los Angeles, CA 90025 (US)
(74) Representative: Pallini Gervasi, Diego
(86) International application number: PCT/US2016/014648
(87) International publication number: WO 2016/118943

(56) References cited:
- WO-A1-01/10358
- US-A- 5 476 434
- US-A1- 2005 216 069
- US-A1- 2006 247 723
- US-A1- 2014 213 979
- US-B1- 6 638 208
- US-B1- 7 147 606
- US-B2- 7 415 308

## Description

### FIELD

This disclosure generally relates to bladder management systems, and in particular, wireless sensors and urinary catheters.

### BACKGROUND

Currently, there are a significant amount of people that suffer from bladder issue where they are unable to sense the amount of urine in their bladder and when they need to urinate. One condition resulting in this symptom is the Neurogenic Bladder, often found in individuals suffering from paralysis. Many of these individuals lack sensation below their levels of injury and this results in someone who Is required to use intermittent catheters to allow urine to empty their bladder. Because they are unable to determine the exact amount of urine stored in their bladder, and unable to sense the fullness, they often utilize a time schedule to ensure they don't experience urinary accidents. This is very inefficient and increases the risk of Urinary Tract Infections, urethral damage due to False Passage, and other issues. If these individuals were able to decrease the number of times they are catheterized, it would improve the individual's comfort and decrease their risk of Infection and other complications, A device that alerts them the amount of urine currently in their bladder can allow individuals to more accurately determine the timing to release the urine stored in their bladder instead of being required to utilize a time schedule. A valve device that is fully internal to the individual's body, which also allows the Individual to open and close the valve from outside the body, would decrease the need to remove and replace the catheter while reducing the likelihood of foreign objects entering the urethra. It Is, therefore, desirable to provide an improved sensor and valve on a catheter, that overcomes most, if not all of the preceding problems.

The US Patent Application Publication No. US 2006/247723 A1 describes a therapeutic sphincter control system with a fluid tube pressure sensor. The system senses sphincter pressure and sends the information to a stimulator that is capable of stimulation therapy to control sphincter contractility, thus reducing unwanted urinary incontinence. Measuring sphincter pressure is accomplished through the use of a fluid-filled tube placed through the sphincter and attached to a module implanted within the bladder. Pressure within the tube is transduced to generate an electrical signal that is sent wirelessly to an implanted stimulator connected to a lead positioned near pelvic floor nerves. According to US 2006/247723 A1 an external device may then be used to wirelessly send information to the implanted stimulator and inhibit stimulation in order for the patient to empty the bladder. Pressure information and stimulation information may be recorded and reviewed for continued patient monitoring.

The US Patent Application Publication No. US 2005/216069 A1 discloses a device for treating a medical condition and a surgical procedure for implanting the device. It includes a sensor, which is adapted to generate a signal responsive to a state of a patient, and at least one electrode, which is adapted to be coupled to a pelvic site of the patient. A control unit is adapted to receive the signal, to analyze the signal so as to distinguish between an imminent stress incontinence event and an imminent urge event, and, responsive to analyzing the signal, to apply an electrical waveform to the at least one electrode.

The US Patent Publication No. US 7 147 606 B1 discloses a diagnostic system comprising a sensing device having a retrieval wire. The sensing device is configured to collect data. The system also includes a deployable housing that has a body defining an interior and an exterior. The housing is configured to allow fluid to flow through the housing and the housing completely encloses the sensing device. The system also includes a disposer for disposing the housing into a body part. The system also includes a processing device configured to receive the data from the sensing device. The processing device is configured to transmit the data.

The US Patent Publication No. US 6 638 208 B1 discloses an implantable apparatus including a plug member having a lumen and a valve adapted to open and close the lumen in response to a signal. The apparatus may also include at least one sensor and a controller adapted to control the valve. The controller may be programmable to open the valve to permit the flow of urine under at least one mode of operation selected from a predetermined time interval operation, a sensor operation, and a manual actuation operation.

### SUMMARY

According to the present invention, it is provided an implantable device for bladder control management in accordance with claim 1.

### BRIEF DESCRIPTION OF THE DRAWINGS

The following drawings and the associated descriptions are provided to illustrate the present disclosure and do not limit the scope of the claims.
FIG, 1 shows a generalized schematic drawing of a sample bladder management system.
FIG. 2 shows a generalized schematic drawing of a sample bladder management system using signal generating sensor.
FIGS., 3 and 4 schematically show different embodiments of sensors used in the bladder management system.
FIG. 5 shows a schematic drawing of a bladder management system used in a male urinary control system.
FIG, 8A shows a schematic drawing of an embodiment of the catheter portion of the bladder management system of FIG. 1.
FIG, 88 shows an enlarged view of a valve portion of the catheter of FIG. 8A.
FIG. 6C shows a cross-section view of the valve portion of FIG. 6B across the line a-b.
FIG. 6D shows an enlarged view of a retaining portion of the catheter of FIG. 8A.
FIG. 7 A shows a schematic drawing of an embodiment of an extraction device.
FIG. 7B shows a schematic drawing of an embodiment of a thruster tip of the extraction device of FIG, 7 A.
FIG. 8A shows a schematic drawing of an embodiment of an insertion device and the catheter.
FIG. 8B shows a schematic drawing of an embodiment of a mating lip of the insertion device of FIG. 8A.
FIG. 9 is a functional block diagram which Illustrates the operation of a sample sound sensor system.

### DETAILED DESCRIPTION

Disclosed herein is an implantable device for bladder control managementin accordance with claim 1 that can be used for bladder management, specifically bladder management for Neurogenic Bladder in a patient. For example, embodiments of the system can be used to sense metrics that can be used to determine the amount of urine inside a person's, or an animal's, bladders and/or the pressure of urine in the bladder. However, the disclosed systems can be used for fluid flow control and sensing for other bodily organs as well and the particular bodily organ described is not limiting. Further, the bladder management can be based on, for example, characteristics of the urine such as pH level, quantity, volume, pressure, urine constituents, color, odor, turbidity, density, possible pathogens, etc. Bladder management can also be based on the dimension of the urethra such as circumference or diameter, pressure of urine Inside the bladder, so forth and the particular example used for bladder management are not limiting. As used herein, the term "user" is intended to include any person trained and able to perform the procedure, including the patient, doctor, caregiver, nurse, etc. The term "patient" and "Individual" are intended to be interchangeable. The term "body" used herein is defined as "an animate body" including human, animal and the like.

### Bladder Management System

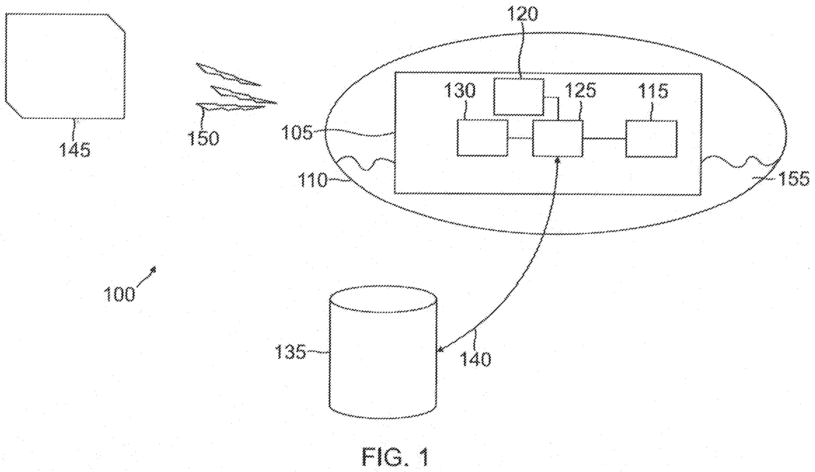

FIG. 1 shows a generalized schematic drawing of a sample bladder management system. A bladder management system 100 comprises a body. The body can be a long-term use catheter 106, The catheter 105 can be shaped and sized to be introduced into the bladder 110 of a patient. The catheter 105 can be fully-Internal to the body of the patient. The device comprises a sensor 115 and a valve 120. The device further comprisesa processor 125 and may comprise a power source 130. Accordig to the present invention, the device comprises a computing device 135, and the processor 125 is configured to communicate with the computing device 135. The processor 125 communicates with the computing device using wireless transmission 140. The computing device 135 can be a mobile phone, in some embodiments, the bladder management system 100 can comprise an external powering system 145. The external powering system 145 can be configured to transmit energy to the catheter 105, For example, the power source 130 can be a battery or capacitor, and the external powering system 145 can charge the power source 130 through inductive or wireless means. The external powering system 145 may also utilize energy in other bands of the spectrum (SONAR, Acoustic, Ultrasound, RF, etc) to transmit data that the external unit can sense to extrapolate information about the condition, volume, pressure, or other characteristics of urine in the bladder and also overall bladder health.

The sensor 115 can be used to measure the level of urine 155 in the bladder 110. The sensor 115 is placed along the body of the catheter 105. The sensor 115 can be inductively and capacitively coupled to the processor 125. The processor 125 is configured to receive an input from the sensor 115 and produce an output that can be used for bladder management. For example, the processor 125 can receive data from the sensor 115, and the output from the processor 125 can be used to alert the user about the characteristics of urine and bladder. This external system may also be used to open or close the valve 120. The valve 120 is in a fluid communication with the catheter 105. The valve 120 is configured to restrict or allow flow of fluid from within the bladder 110. For example, the valve 120 can be positioned within a portion of the catheter 105 along the urethra. The power source 130 can be inductively and capacitively coupled with the processor 125 and/or the external powering system 145. The power source 130 can also be coupled with the valve 120. The power source 130 can be used, for example, to supply the processor with the power to send or receive Information from the computing device 135. The power source 130 can be coupled with the valve 120 and be used to supply the power required to open or close the valve 120, The external powering system 145 can be connected to an AC outlet and/or utilize DC current from other sources.

The power source 130 can he rechargeable. The power source 130 can be configured to last for an approximate 1-38 months of usage, in some embodiments, the power source 130 is a battery that can be configured to receive an electric charge from an external powering system 145 via wireless recharging technology similar to what is currently available in other devices as Commercial Off The Shelf (COTS) application for wireless induction charging.

The external powering system 145 can comprise a charging pod. The charging pod can be plugged into an alternating current (AC) outlet. When placed in the proscribed location, such as on the front of the abdomen, the power source 130 in the catheter 105 can recharge through magnetic induction technology, for example.

In some embodiments, the computing device 135 can have a software which can be used to interpret the values sent from the sensor 115. In some embodiments, the sensor 115 is a pressure sensor and the computing device 135 can be used to alert a user about when their bladder is likely to contract and void. In some embodiments, the urine 155 amounts inside the bladder can be calibrated by feedback from the Individual user after insertion or implantation. In some embodiments, the sensor can use other spectrums of energy, to include acoustics, to determine the fullness or volume of urine In the bladder. Different types of sensors can be embedded on the catheter to determine important metrics of bladder health including pH, volume, pressure, etc. In some embodiments, this can be accomplished through software that analyzes the sensor 115 response and utilizes machine learning algorithms to predict and interpret this data.

The sensor 115 device can utilize basic wireless transmission protocol to wirelessly send data to a computing device 135 with the control software on it. This can be accomplished in a manner similar to Bluetooth, 802.11 Wifi, SONAR, UltraSound, MedRadio or other wireless communications protocols.

In one embodiment the sensor 115 can determine the pressure of urine 155 within the bladder 110 and send a signal to the processor 125. The processor 125 sends information on pressure level in the bladder 110 to the computing device 135. The computing device 135, using a software, determines whether urine 155 needs to be drained from the bladder 110. The computing device 135 will also notify the user to drain urine from the bladder. The user can actuate the valve 120 which will allow urine 155 to leave the bladder 110. In some embodiments, the sensor 115 can be used to determine when urine has been sufficiently drained from the bladder 110, such as by determining that the pressure level within the bladder 110 has dropped below a certain level. This Information can be used to close the valve 120 and halt the flow of urine 155 from leaving the bladder 110. This sensing technology is not limited to pressure, and in some embodiments, other metrics can be used to make decisions with clinical impact.

The catheter 105 can be used to determine various conditions within the bladder 110. The sensor 115 can be a pH sensor, an ultrasonic sensor, a displacement sensor, acoustic sensor, etc. Different types and combinations of sensors can be used. For example, the catheter 105 can comprise a pH sensor and a pressure sensor.

The valve 120 can be configured to increase or decrease the flow rate of urine. For example, the valve can have varying degree of valve opening. The valve 120 opening can be configured to dilate and/or expand in order to increase the volumetric flow rate of urine leaving the bladder 155. For example, a pin valve can be used. This valve 120 can also be configured to be actuated by the pressure of urine 155 in the bladder 110.

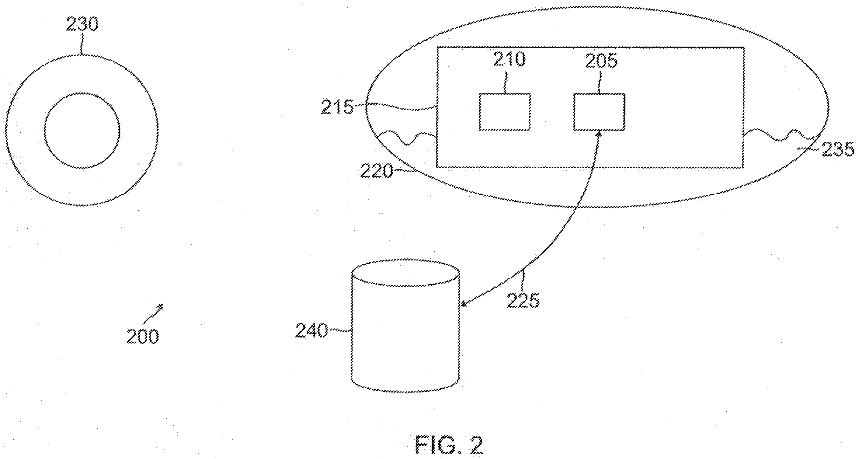

As shown in FIG. 2, a bladder management system 200 can comprise a signal- generating sensor 205. In some embodiments, the bladder management system 200 can comprise a valve 210, an external actuator 230, the signal-generating sensor 205 within a catheter 215, and an external computing device 240. The signal generating sensor 205 can be an LC resonant sensor, an FID device, or a speaker. In some embodiments, the system 200 can further comprise a microphone and/or an amplifier. The valve 210 can be configured to open and close using an external valve actuator 230. The external computing device 240 can be used to send and receive signals 225 to and from the signal- generating sensor 206. In some embodiments, the external computing device 240 can be programmed to interpret the signal 225 to indicate condition within a patient's bladder 220.

The user can use the external computing device 240 to generate a signal 225. The sensor 206 can receive the signal 225 from the external computing device 240 and generate a return signal 225 that can be analyzed and interpreted to determine metrics about the condition of the bladder and/or urine within the bladder (e.g. volume, pressure, pH level, etc.), The computing device 240 can display the desired information to the user or can be cataloged for further review and analysis. In some embodiments, this data may be used to send an alert or notify the user or caregiver based on either pre-determined settings or through machine learning algorithms and/or advanced data analysis techniques. For example, the computing device 240 can receive signal from the sensor 205 that can be interpreted to determine the volume of urine 235 in the bladder 220. Another example, an algorithm can be used to determine the pressure within the bladder 220 based on relationships with the volume of the bladder 220. Based on the information available or the notification provided, the user can manually open the valve 210 using the external actuator 230.

The sensor 205 can be configured to change its mechanical properties (e.g. color, size, shape, etc.) based on pressure changes inside the bladder 220. The user can use an external device to detect changes in mechanical properties of the sensor 205 by, for example, sending and/or receiving sound waves, light waves, etc. In some embodiments, the internal sensor 205 can function without a power source. By constructing the sensor 205 in a specific manner the external unit can observe changes in the resonant frequency characteristics.

The catheter 215 can comprise a computing device 240. For example, a computing device 240 can be coupled with the sensor 205 and use a software algorithm to determine urine 235 amount inside the bladder 220 based on data read from the sensor 205. The user can use an external device that can exchange information with the computing device 240 coupled with the sensor 205, In some embodiments, the computing device 240 can be linked to an external database. For example, an external database having patient data can be used with the computing device 240 to monitor the patient condition and manage bladder 220 by using data that has been collected and aggregated from other patients and sources. A database can catalog the dataset that will allow for analysis and algorithm development to improve accuracy and support predictive analytics.

### Sensor

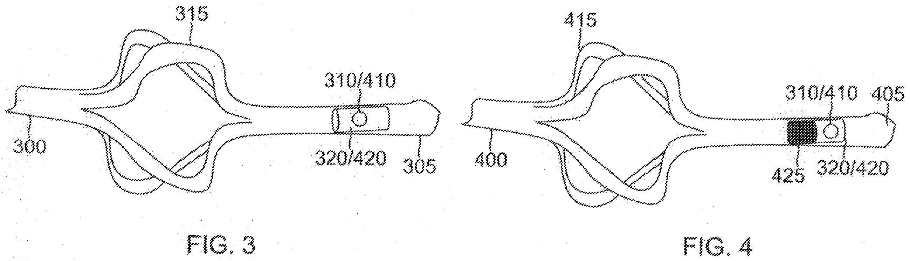

As schematically shown in FIGS. 3 and 4, sample embodiments of the device can comprise a catheter 300, 400 having a proximal end 306, 405. The catheter 300, 400 can comprise a lumen 310, 410 having a retaining portion 315, 415 near the proximal end 305, 405. The catheter 300, 400 can house a sensor 320, 420. As shown in FIG. 4, the sensor 420 can comprise a radio unit 425. For example, the radio unit 425 can be a transceiver. In some embodiments, the radio unit 425 can comprise a microphone. The radio unit 425 can be wirelessly coupled to the sensor 420 via different means, including inductive-capacitive coupling or energy transmission in other mediums such as SONAR, UltraSound, Microwave, etc.

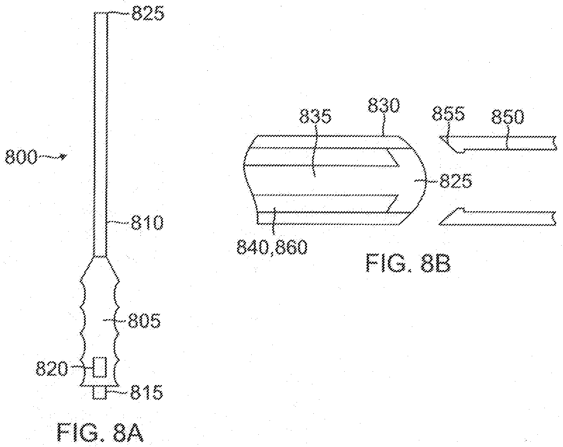

The retaining portion 315, 415 can be configured to transition from an expanded configuration as shown in FIGS. 3-4 to a collapsed configuration shown in FIG. 8A. The retaining portion 315, 415 can be a malecot anchor having a plurality of wings. In the expanded configuration, the retaining portion 315, 415 can retain and anchor the catheter 300, 400 within the bladder. In the collapsed configuration, the catheter can be received within and passed through the bladder neck and urethra without causing trauma to the urethra or significant modification to the human anatomy.

The catheter 300, 400 which includes the sensor 320, 420 can perform one or more of the following: measure important metrics of urine and the bladder; wirelessly relay this data to an external device; allow a mechanical valve to open upon user input, be semi-permanent, e.g. allowing long-term use and/or extended wear; be inserted via minimally invasive means: utilize wireless recharging or powering technology for the sensor 320, 420 or power storage unit; and be removed when the user wishes to remove the catheter.

### Catheter

As shown in FIG, 5, the catheter 500 can be fully-internal, meaning the catheter 500 is not visible to the naked eye from the exterior, once the catheter 500 is inside the patient's body. The fully-internal catheter 500 can comprise a proximal portion 505 and a distal portion 510. The proximal portion 505 can comprise a retaining portion 515 and a sensor 520. The catheter 500 can comprise a lumen 525. The distal portion 510 can comprise a valve 530.

The sensor 520 can be placed proximal to the retaining portion 515. The sensor 520 can be configured to communicate with an external computing device 535, In some embodiments the computing device 535 is a mobile phone. In some embodiments, the external computing device 535 may be a transceiver that is able to receive and relay these signals to other devices. The valve 530 can comprise a mating structure 540. The mating structure 540 can be configured to mate with a corresponding structure of a catheter insertion device and/or that of an extraction device.

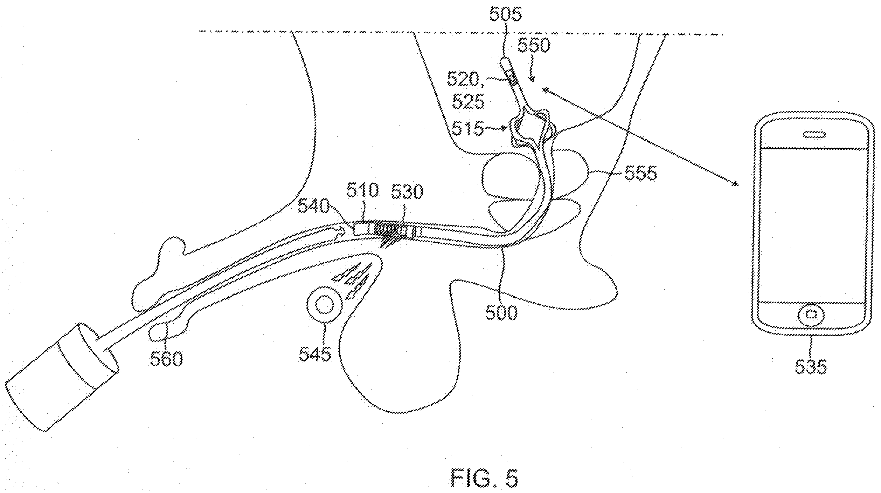

In some embodiments, the urine in the bladder 550 can be voided when the user utilizes an external actuator 545 to open the valve 530 and allows the urine to travel through the urethra 560. This signal can be controlled by the user through the use of an external computing device 535. In some embodiments, the valve 530 can comprise a magnetic ball valve, and the external actuator 545 can comprise a magnet. In other embodiments, this external actuator 545 can be a combination of electronic control that may utilize an electromagnet to open the valve 530. As show in FIG. 5, a user can place the external actuator 545 near the valve 530, e.g. on the skin of patient between the scrotum and the shaft of the penis, to open the valve 530. The valve 530 can be closed when the actuator 545 is away from the location of the valve 530 inside the patient's body. For example, the valve 530 can be closed when the actuator is inside a patient's side pocket.

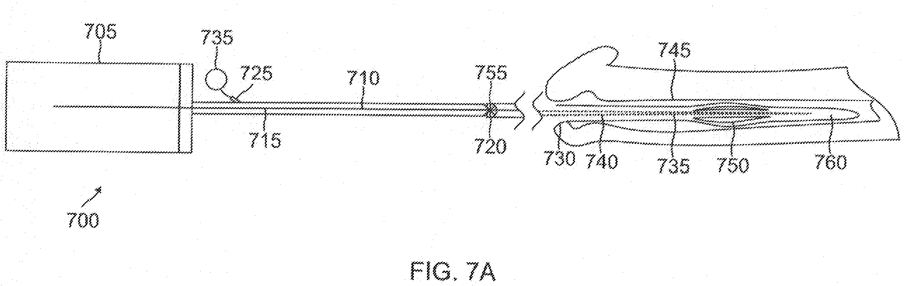

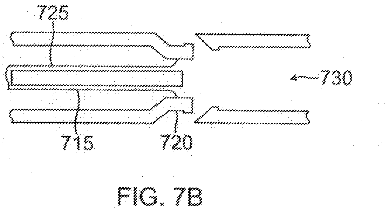

The catheter 500 can be constructed in a shape and of a material that is conducive to entry utilizing a medical device, such as an insertion device 700 show in FIG 7A and 7B, that will enter through the urinary tract. For example, the catheter 500 may be constructed of a material similar to other existing intermittent catheters on the market (such as PVC, Latex, Silicone, Polyurethane or any blend of these materials). In some embodiments, once inside the bladder 550, the catheter 500 can comprise a retaining portion 515 that can fix the catheter 500 to the wall of the bladder 550.

in some embodiments, the sensor 520 can be placed distal to the retaining portion 515. For example, the sensor 520 can be coupled to the valve 530. In some embodiments, the valve 530 can comprise an internal actuator. For example, the valve 530 can be configured such that an external computing device 535 can be used to open or close the valve 530 using a signal. In some embodiments, a valve 530 can be placed in the proximal portion 505 of the catheter. For example, the valve 530 can be placed on the neck of the bladder 550. In some embodiments, the catheter 500 can comprise a retaining portion 515 along a midsection or distal end of the body. For example, the catheter 500 can be configured such that the retaining portion 515 is placed on or near the prostate 555 or along the urethra 560 of the patient, instead of within the bladder 550 of the patient as shown. In some embodiments, this mechanism may be used in combination with the malecot anchor.

### Valve

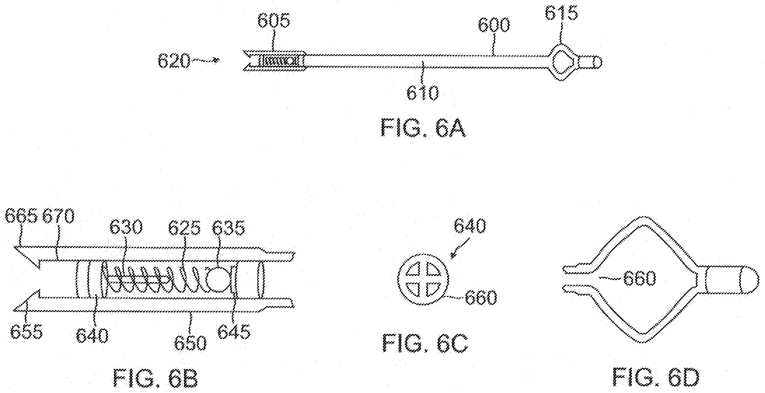

FIGS, 6A to 6D are detailed schematic drawings of the catheter 600 using a ball valve 605. As shown in FIG, 5A, the catheter 600 comprises an elongated midsection 610 between the proximal portion 615 and the distal portion 620. The elongated midsection 610 can comprise a flexible tube having a lumen. As shown in FIG 6B, the valve 605 can be a cylindrical magnetic bail valve comprising a spring 625, a spring stabilizer 630, a magnetic ball 635, a distal seat 640. a proximal seat 645, and a mating structure 855. The proximal portion 615 can comprise a straight tip. In some embodiments, the proximal portion 615 comprises a coude tip.

The spring 825 can be coupled to the distal seat 640 and configured to constantly exert tension during opening and closing of the valve 605. The spring stabilizer 630 can be concentric to the center of the valve 605. The proximal seat 645 can be configured to trap the ball 635 (e.g. by having a ring shape with an inner radius smaller than the radius of the ball 635). The retainer 650 can be positioned distal and adjacent to the proximal seat 645. As shown in FIG 6C, the distal seat 640 and the proximal seat 645 can comprise one or more inlets 660. The inlet 660 can be shaped and sized to pass a pushwire 715 shown and described below in reference to FIGS 7A and 7B. As shown in FIG 6D, the proximal portion 615 can comprise a fluid inlet 660. The proximal portion 615 can house a sensor. The mating structure 655 can comprise a tapered surface 665 and a ledge 670. The mating structure 655 can be configured to expand when thrusting a rigid corresponding structure, e.g. the thruster tip 825 of the extraction device 800 shown in FIG 8. The mating structure 655 can comprise a magnetic material. In some embodiments, the mating structure 655 can comprise a conductive material that can be used to complete an external circuit and inform a user that the extraction device 800 is mated to the mating structure 830.

In some embodiments, the valve 605 can comprise a disc, plug, diaphragm, pinch, check, plunger, flap, duckbill, or other valve designed to actuate upon user input and/or at a calculated pressure threshold. In some embodiments, the valve 605 can comprise a manual squeeze valve (not shown) configured to manually open and close. The manual squeeze valve can comprise an elastic body in a closed state without manual adjustment. When the catheter 600 is inside the patient, the user may squeeze the valve 605 from the skin of the patient to open the valve 605. From squeezing the valve 605. the fluid inside the bladder can be drained.

### User Insertion and Removal

A fully-internal catheter 600 can be used for extended periods and long-term use rather than requiring intermittent replacement as some existing catheter devices require. The long-term use of a fully-internal catheter 600 can aid in patient comfort, prevent and/or reduce psychological trauma from frequent replacement, reduce occurrence of urinary tract infections, etc. The catheter 600 device can be long-term use because it can be inserted and/or removed by utilizing medical devices, e.g. an insertion device 700, an extraction device 800. etc. The removal of the long-term use catheter 600 device can be in a similar manner as when the long-term use catheter 600 device is implanted within the patient. The insertion device 700 and the extraction device 800 can comprise structures corresponding to the mating structure 655 of the valve 605. The device has been designed in such a way that insertion and extraction can be completed by the user.

### Insertion

As shown in FIG 7A, the insertion device 700 can comprise a container 705, an insertion rod 710, a pushwire 715, a trigger 735, and one or more pull strings 725. The pushwire 715 can be a mandrel. As schematically shown in FIG 7B, the insertion rod 710 further comprises a lip 720 connected to the one or more pull strings 725. The lip 720 can be configured to mate with the valve mating structure 830. For example, as shown in FIG 7B, the tip 720 can comprise a bottleneck structure that mates with the ledge 845 of the mating structure 830. When prepared for insertion, the insertion device 700 will translate force along the axis of travel through the urethra 745 until the user chooses to disengage the mating mechanism. The lip 720 can be semi-rigid and have a stiffness sufficient to remain attached to the valve 755 mating structure 830 until disengaged by the user.

The container 705 can be in fluid communication with the rod 710. The container 705 can be configured to allow visual confirmation of material inside the container 705. For example, the container 705 can comprise a translucent material, such as translucent PVC. The rod 710 can comprise a hollow tube made of a medical grade material, such as nylon. The pushwire 715 can pass through the hollow tube of the rod 710 and the catheter 740 lumen and comprises a shape memory material used in similar medical applications. For example, the pushwire 715 can comprise a Teflon^{®}-coated nitinol or stainless steel wire having a stiffness to allow bending and flexing without causing trauma to the urethra 745 while the catheter 740 is inserted inside the patient's body. The pushwire 715 can have a longitudinal length longer than the combined longitudinal length of the catheter 740 and the rod 710. As shown in FIG 7A, the pushwire 715 has a length such that a portion of the pushwire 715 extends into the container 705, while the pushwire 715 extends fully along the erect length of the catheter 740 and the rod 710. During insertion, the pushwire 715 contacts the catheter tip 760 such that thrusting force from the pushwire 715 transports the catheter 740 along the urethra and to the bladder. In some embodiments, the trigger 135 can comprise a ring shape having a dimension to fit a human index finger. The trigger 735 can be connected to the pull string 725 which extends from the lip 720 and through an opening on the rod 710.

The insertion device 700 and the catheter 740 can be carried in a sterilized pouch, In some embodiments, the insertion device 700 and the catheter 740 can be in a mated state before use. In the mated state, the pushwire 715 extends through the valve 755 such that the valve 755 remains open to allow fluid flow. As shown in FIG 7A, the retaining portion 750 of the catheter 740 can be folded in the mated state. A user may open the pouch comprising a catheter 740 and the insertion device 700 and insert the catheter 740 into the urethra. The user may move the catheter 740 by moving the catheter 740 proximally using the insertion device 700. Once the retaining portion 750 reaches the bladder, the retaining portion 750 can expand as the user removes the pushwire 715 and allows the anchor to return to its resting state. The fluid within the bladder may drain from an opening 730 of the retaining portion 750 through the open valve 755, to the rod 710, and eventually to the container 705. The user may observe presence of fluid inside the container 705 to visually confirm placement of catheter 740 and that the catheter 740 has successfully reached the bladder. The user can then remove the pushwire 715 and allow the anchor to expand. The user can actuate the trigger 735 to collapse the tip 720 to disconnect the insertion device 700 from the catheter 740. The removal of the insertion device 700 can be done subsequent to visual confirmation of the placement of catheter 740. The user can move the insertion device 700 away from the catheter 740 while the catheter 740 remains within the patient's body.

In some embodiments, fluid flow through the valve 755 can be prevented before and after the insertion of the catheter 740. For example, the valve 755 can comprise an orifice completely sealed off by the pushwire 715 to prevent fluid flow. In some embodiments, the catheter 740 can comprise a sensor which can notify the user of fluid flow in the catheter 740 upon placing the retaining portion 750 inside the bladder.

### Extraction Device

As shown in FIG 8A, the extraction device 800 can comprise a handle 805, an extraction rod 810, a click button 815. and a visual indicator 820. The extraction rod 810 can comprise a cannula. As shown in FIG 8B. the extraction device 800 can comprise a thruster 835 inside the extraction rod 810. The thruster 835 can comprise an extraction tip 825. The extraction tip 825 can be configured to mate with the mating structure 830 of the valve 755. For example, the extraction tip 825 can comprise a semi-rigid surface 860 that can be pushed past the tapered surface 855, and one or more latching wings 840 that can latch onto the ledge 850. The extraction tip 825 can comprise a magnetic material. In some embodiments, the extraction tip 825 can comprise a conducting material. The click button 815 can be located on the distal tip of the handle 805 opposite the extraction rod 810. The visual indicator 820 can be an LED light configured to actuator on or off when the extraction tip 825 abuts the valve mating structure 830. The visual indicator 820 can be located on the handle 805

The handle 805 can comprise an ergonomic structure and can house a spring. The click button 815 can be used to operate the thruster 835 from a protruding position and retracting position, having mechanics similar to a conventional retractable pen. The extraction device 800 can provide auditory and tactile notice to the user, such as when the click button is pressed, the button "clicks" to indicate that the thruster 835 position has changed. In some embodiments, the handle 805 and the extraction rod 810 can house electronic circuitry connected to the visual indicator. The electronic circuitry connected to the visual indicator 820 can remain broken until the conductive thruster tip 825 connected to the circuitry contacts the valve mating structure 830 to complete the circuitry. For example, the valve mating structure 830 can comprise an annular conductive surface, while the extraction tip 825 can comprise two or more disconnected probe ends configured to contact the annular conductive surface.

The extraction rod 810 of the extraction device 800 can be inserted in the urethra of a patient wearing the fully-internal catheter. The user can determine placement of the extraction tip 825 to the valve mating structure 830 by seeing the visual indicator tight turn on. The extraction tip 825 and the valve mating structure 830 can magnetically attach. The user may push the click button 815 to push the latching wing 840 of the thruster tip 825 past the ledge 850. The thruster tip 825 can flex and collapse to push through the tapered surface 855 and contract to its original shape as the latching wing 840 moves past the ledge 850 to latch onto the ledge 850. The user may move the extraction device 800 to extract the catheter out of the patient's body and dispose the extraction device 800 and the catheter.

In some embodiments, the visual indicator 820 can remain on until the thruster tip 825 contacts the valve mating structure 830. In some embodiments, the visual indicator 820 can be located on the click button 815. Various different types of actuation mechanism can be used. For example, the thruster 835 can be actuated using a turn knob or a screw. In some embodiments, the click button 815 can be located on the side of the handle 805.

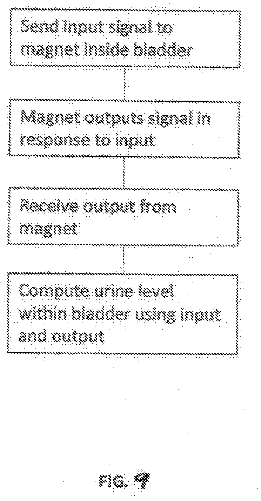

FIG. 9 is a functional block diagram which illustrates the operation of a sample sound sensor system.

Among the many advantages of this invention include increasing the quality of life for individuals suffering from neurogenic bladder by: 1. reducing the risk of medical issues (urinary tract infections, false passage, etc.); 2. eliminating the need for indwelling or intermittent catheters and decreasing the number of catheters required for daily use (because of increased accuracy with which the user knows when catheterization is required); 3. allowing the user to control bladder voiding; 4, accommodating implanted, semi-permanent (useful life 3-6 months) device via minimally invasive means (via catheter); 5. minimizing problems from incontinence and related psychological impact (emotional trauma from accidental urinary voiding); and 6. transmits wireless report data similar to that done in urodynamic flow testing (pressure of bladder at different levels of fullness) more accurately and less invasively.

Although embodiments of the invention have been shown and described, it is to be understood that various modifications, substitutions, rearrangements and different parts, components, equipment, elements and/or process (method) steps, as well other uses, of the wireless pressure sensor and valve for bladder can be made by those skilled in the art without departing from the scope of this invention which is only limited by the appended claims.

While several embodiments of the present disclosure have been described and illustrated herein, those of ordinary skill in the ail will readily envision a variety of other means and/or structures for performing the functions and/or obtaining the results and/or one or more of the advantages described herein, and each of such variations and/or modifications is deemed to be within the scope of the present disclosure. Mom generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

As used In any embodiment herein, the term "module" may refer to software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices. "Circuitry", as used in any embodiment herein, may comprise, for example, singly or in any combination, hardwired circuitry, programmable circuitry such as computer processors comprising one or more individual instruction processing cores, state machine circuitry, and/or firmware that stores Instructions executed by programmable circuitry. The modules may, collectively or individually, be embodied as circuitry that forms part of a larger system, for example, an Integrated circuit (IC), system on-chip (SoC), desktop computers, laptop computers, tablet computers, servers, smart phones, etc.

Any of the operations described herein may be Implemented in system that includes one or more storage mediums having stored thereon, individually or in combination, instructions that when executed by one or more processors perform the methods. Here, the processor may include, for example, a server CPU, a mobile device CPU, and/or other programmable circuitry.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as If it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language In the specification should be construed as indicating any non-daimed element essential to the practice of the invention.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Specific embodiments disclosed herein may be further limited in the claims using consisting of or consisting essentially of language. When used in the claims, whether a filed or added per amendment, the transition term "consisting of excludes any element, step, or ingredient not specified in the claims. The transition term "consisting essentially of" limits the scope of a claim to the specified materials or steps and those that do not materially affect the basic- and novel characteristic(s). Embodiments of the invention so claimed are inherently or expressly described and enabled herein.

Furthermore, numerous references have been made to patents and printed publications throughout this specification.

in closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described,

Unless otherwise indicated, all numbers expressing quantities of ingredients, properties such as molecular weight, reaction conditions, and so forth used in the specification and claims are to be understood as being modified in all instances by the term "about". Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessary resulting from the standard deviation found in their respective testing measurements.

The terms "a," "an," "the" and similar referents used in the context of describing the Invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

M Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

As one skilled in the art would recognize as necessary or best-suited for performance of the methods of the invention, a computer system or machines of the invention include one or more processors (e.g., a central processing unit (CPU) a graphics processing unit (GPU) or both), a main memory and a static memory, which communicate with each other via a bus.

A processor may be provided by one or more processors including, for example, one or more of a single core or multi-core processor (e.g., AMD Phenom II X2, Intel Core Duo. AMD Phenom II X4, Intel Core i5, Intel Core I & Extreme Edition 980X, or Intel Xeon E7-2820).

An I/O mechanism may include a video display unit (e.g., a liquid crystal display (LCD) or a cathode ray tube (CRT)), an alphanumeric input device (e.g., a keyboard), a cursor control device (e.g., a mouse), a disk drive unit, a signal generation device (e.g., a speaker), an accelerometer, a microphone, a cellular radio frequency antenna, and a network interface device (e.g., a network interface card (NIC), Wi-Fi card, cellular modem, data jack, Ethernet port modem jack, HDMI port, mini-HDMI port, USB port), touchscreen (e.g., CRT, LCD, LED, AMOLED, Super AMOLED), pointing device, trackpad, light (e.g., LED), light/image projection device, or a combination thereof.

Memory according to the invention refers to a non-transitory memory which is provided by one or more tangible devices which preferably Include one or more machine-readable medium on which is stored one or more sets of instructions (e.g., software) embodying any one or more of the methodologies or functions described herein. The software may also reside, completely or at least partially, within the main memory, processor, or both during execution thereof by a computer within system, the main memory and the processor also constituting machine-readable media. The software may further be transmitted or received over a network via the network interface device.

While the machine-readable medium can in an exemplary embodiment be a single medium, the term "machine-readable medium" should be taken to include a single medium or multiple media (e.g.. a centralized or distributed database, and/or associated caches and servers) that store the one or more sets of instructions. The term "machine-readable medium" shall also be taken to include any medium that is capable of storing, encoding or carrying a set of instructions for execution by the machine and that cause the machine to perform any one or more of the methodologies of the present invention. Memory may be, for example, one or more of a hard disk drive, solid state drive (SSD), an optical disc, flash memory, zip disk, tape drive, "cloud" storage location, or a combination thereof. In certain embodiments, a device of the invention includes a tangible, non-transitory computer readable medium for memory. Exemplary devices for use as memory include semiconductor memory devices, (e.g., EPROM. EEPROM, solid state drive (SSD), and flash memory devices e.g., SD. micro SD. SDXC, SDIO, SDHC cards); magnetic disks, (e.g., internal hard disks or removable disks); and optical disks (e.g., CD and DVD disks).

Furthermore, numerous references have been made to patents and printed publications throughout this specification. Each of the above-cited references and printed publications are individually incorporated herein by reference in their entirety.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized In accordance with the teachings herein.

## Claims

1. An implantable device for bladder control management, the implantable device comprising:
a body having a proximal end and a distal end and configured to be implanted within a bladder of a patient
at least one pressure sensor (115, 320, 420, 520) positioned along a portion of the body, the pressure sensor (115) configured to generate a plurality of pressure measurement signals as functions of pressure imparted upon the pressure sensor (115, 320, 420, 520);
a processor (125) mounted on the body and electrically coupled to the at least one pressure sensor (115, 320, 420, 520), the processor (125) configured to wirelessly transmit the pressure measurement signals to an external computing device (240, 535) for subsequent output of a level of urine within the bladder based on one or more of the pressure measurement signals;
a retaining member positioned along a portion of the body, the retaining member configured to transition between an expanded configuration to retain the implantable device within the bladder and a collapsed configuration to allow the implantable device to be received within and passed through the bladder neck and urethra, and wherein the proximal end of the implantable device comprises an attachment mechanism configured to engage a corresponding attachment mechanism on a positioning catheter for positioning and retrieving the implantable device;
wherein a portion of the body comprises a lumen (310, 410, 525) extending towards the proximal end and is configured to allow fluid to flow therethrough, the lumen (310, 410, 525) is in fluid communication with an opening defined along a portion of the body;
a valve member (120, 210, 530, 605, 630, 755, 805) in fluid communication with the lumen (310, 410, 525) and electrically coupled to the processor (125); and
wherein, upon receiving input from the external computing device (240, 535), the processor (125) is configured to control operation of the valve (120, 210, 530, 605, 630, 755, 805) to transition between an open position and a closed position, and any number of positions there between, to thereby control flow of fluid through the lumen (310, 410, 525).

2. The implantable device of claim 1, further comprising a power source (130) coupled to the processor (125) and configured to provide power to at least the pressure sensor (115, 320, 420, 520).

3. The implantable device of claim 2, wherein the power source (130) comprises a battery.

4. The implantable device of claim 3, wherein the battery is rechargeable, preferably wherein the processor (125) is configured to communicate with an external recharging system and wirelessly receive energy therefrom to thereby charge the battery.

5. The implantable device of claim 4, wherein the processor (125) comprises inductive charging components.

6. The implantable device of claim 2, further comprising one or more leads extending from the body and electrically coupled to the processor (125) and power source (130).

7. The implantable device of claim 6, wherein, upon receiving input from the external computing device (240, 535), the processor (125) is configured to transmit an electrical signal from the power source (130) to the one or more leads, wherein at least one of the leads is configured to contact an internal portion of the bladder and transmit the electrical signal thereto to cause electrical stimulation and contraction of the bladder.

8. The implantable device of claim 6, wherein at least one of the leads comprises a distal end configured to engage an internal portion of the bladder to secure positioning of the implantable device within the bladder, preferably wherein the distal end comprises a hook member configured to attach to an internal wall of the bladder.

9. The implantable device of claim 1, wherein the proximal end of the implantable device comprises an attachment mechanism configured to engage a corresponding attachment mechanism on a positioning catheter for positioning and retrieving the implantable device and wherein, upon
(i) engagement between the attachment mechanisms, the retaining member is configured to transition to the collapsed configuration; or
(ii) disengagement of the attachment mechanisms from one another, the retaining member is configured to transition to the expanded configuration.

10. The implantable device of claim 1, wherein:
(i) at least the body of the implantable device is comprised of a medical grade material; or
(ii) the body of the implantable device has a shape or geometry conducive to being delivered into the bladder via an endoscopic device.

11. The implantable device of claim 1, wherein the processor (125) is configured to wirelessly transmit data via a wireless transmission protocol selected from the group consisting of: Bluetooth communication, infrared communication, near field communication (NFC), radio-frequency identification (RFID) communication, cellular network communication, the most recently published versions of IEEE 802.11 transmission protocol standards as of April 2015, and a combination thereof.

## Patentansprüche

1. Implantierbare Vorrichtung zur Steuerung der Blasenentleerung, wobei diese implantierbare Vorrichtung umfasst:
einen Körper, welcher ein proximales Ende und ein distales Ende aufweist und welcher dergestalt ausgelegt ist, dass er in die Blase eines Patienten implantiert werden kann;
mindestens einen Drucksensor (115, 320, 420, 520), welcher längs eines Teils dieses Körpers angeordnet ist, wobei dieser Drucksensor dergestalt ausgelegt ist, dass er eine gewisse Anzahl von Druckmesssignalen erzeugt, welche Funktionswerte für den Druck darstellen, der auf den Drucksensor (115, 320, 420, 520) ausgeübt wird;
einen Prozessor (125), welcher auf den Körper montiert ist und elektrisch an den mindestens einen Drucksensor (115, 320, 420, 520) angeschlossen ist, wobei dieser Prozessor (125) dergestalt ausgelegt ist, dass er die Druckmesssignale drahtlos an einen externen Computer (240, 535) überträgt zum Zweck der nachfolgenden Ausgabe eines Pegelwerts für den Urin in der Blase auf der Grundlage von einem oder mehreren der Druckmesssignale;
ein Rückhalteglied, welches längs eines Teils des Körpers angebracht ist, wobei dieses Rückhalteglied dergestalt ausgelegt ist, dass es aus einem expandierten Zustand, in welchem die implantierbare Vorrichtung in der Blase zurückgehalten wird, in einen zusammengefallenen Zustand übergeht, um dadurch zu ermöglichen, dass die implantierbare Vorrichtung im Blasenhals Aufnahme findet und durch den Blasenhals und die Harnröhre hindurchgeführt wird, und bei welchem das proximale Ende der implantierbaren Vorrichtung einen Befestigungsmechanismus umfasst, welcher dergestalt ausgelegt ist, dass er mit einem entsprechenden Befestigungsmechanismus auf einem Positionierkatheter in Verbindung kommt, um die implantierbare Vorrichtung zu positionieren und wieder zu entfernen;
wobei ein Teil des Körpers einen Hohlraum (310, 410, 525) umfasst, welches sich in Richtung auf das proximale Ende erstreckt und dergestalt ausgelegt ist, dass ermöglicht wird, dass Flüssigkeit hindurchströmt, wobei dieser Hohlraum strömungsmäßig mit einer Öffnung in Verbindung steht, die längs eines Teils des Körpers festgelegt ist;
ein Ventilelement (120, 210, 530, 605, 630, 755, 805) in strömungsmäßiger Verbindung mit dem Hohlraum (310, 410, 525) und mit elektrischem Anschluss an den Prozessor (125);
wobei nach Eingang des Eingangssignals von dem externen Computer (240, 535) der Prozessor (125) dergestalt konfiguriert wird, dass er den Betrieb des Ventils (120, 210, 530, 605, 630, 755, 805) steuert, damit dieses aus einem offenen Zustand in einen geschlossenen Zustand und umgekehrt mit einer Anzahl von Zwischenzuständen übergeht, um dadurch die Strömung der Flüssigkeit durch den Hohlraum (310, 410, 525) zu steuern.

2. Implantierbare Vorrichtung nach Anspruch 1, welche außerdem eine Energiequelle (130) umfasst, die an den Prozessor (125) angeschlossen ist und dergestalt ausgelegt ist, dass sie die Energie für den mindestens einen Drucksensor (115, 320, 420, 520) liefert.

3. Implantierbare Vorrichtung nach Anspruch 2, bei welcher die Energiequelle (130) eine Batterie umfasst.

4. Implantierbare Vorrichtung nach Anspruch 3, bei welcher die Batterie wieder aufladbar ist und bei welcher vorzugsweise der Prozessor (125) dergestalt ausgelegt ist, dass er mit einem externen Wiederaufladesystem in Verbindung steht und daraus auf drahtlose Weise Energie aufnimmt, um damit die Batterie zu laden.

5. Implantierbare Vorrichtung nach Anspruch 4, bei welcher der Prozessor (125) Bauteile zum induktiven Aufladen umfasst.

6. Implantierbare Vorrichtung nach Anspruch 2, welche außerdem eine oder mehrere Leitungen umfasst, welche sich vom Körper aus erstrecken und elektrisch an den Prozessor (125) und die Energiequelle (130) angeschlossen sind.

7. Implantierbare Vorrichtung nach Anspruch 6, bei welcher nach Eingang des Eingangssignals von dem externen Computer (240, 535) der Prozessor (125) dergestalt konfiguriert wird, dass er ein elektrisches Signal von der Energiequelle (130) an die eine oder die mehreren Leitungen überträgt, wobei mindestens eine der Leitungen dergestalt ausgelegt ist, dass sie mit einem inneren Teil der Blase in Kontakt kommt und das elektrische Signal dorthin überträgt, um die elektrische Stimulation und Kontraktion der Blase zu bewirken.

8. Implantierbare Vorrichtung nach Anspruch 6, bei welcher mindestens eine der Leitungen ein distales Ende umfasst, welches dergestalt ausgelegt ist, dass es mit einem inneren Teil der Blase in Kontakt kommt, um die Positionierung der implantierbaren Vorrichtung im Innern der Blase abzusichern, wobei vorzugsweise das distale Ende ein Hakenelement umfasst, welches dergestalt ausgelegt ist, dass es an einer Innenwand der Blase festsitzt.

9. Implantierbare Vorrichtung nach Anspruch 1, bei welcher das proximale Ende der implantierbaren Vorrichtung einen Befestigungsmechanismus umfasst, welcher dergestalt ausgelegt ist, dass er sich mit einem entsprechenden Befestigungsmechanismus an einem Positionierkatheter verbindet zum Zweck der Positionierung und des Entfernens der implantierbaren Vorrichtung und bei welcher
(i) nach dem Zustandekommen der Verbindung zwischen den Befestigungsmechanismen das Rückhalteglied dergestalt konfiguriert wird, dass es in den zusammengefallenen Zustand übergeht; oder
(ii) nach dem Trennen der Befestigungsmechanismen das Rückhalteglied dergestalt konfiguriert wird, dass es in den expandierten Zustand übergeht.

10. Implantierbare Vorrichtung nach Anspruch 1, bei welcher
(i) zumindest der Körper der implantierbaren Vorrichtung aus einem für medizinische Zwecke geeigneten Material besteht oder
(ii) der Körper der implantierbaren Vorrichtung eine Gestalt oder Geometrie aufweist, die dazu dienlich ist, dass sie in die Blase mittels einer endoskopischen Vorrichtung eingeführt werden kann.

11. Implantierbare Vorrichtung nach Anspruch 1, bei welcher der Prozessor (125) dergestalt ausgelegt ist, dass er die Daten drahtlos über ein drahtloses Übertragungsprotokoll überträgt, welches aus der Gruppe ausgewählt wird, die aus folgenden Komponenten besteht: Bluetooth-Kommunikation, Infrarot-Kommunikation, Nahfeld-Kommunikation (NFC), Identifizierung mittels elektromagnetischer Wellen (RFID), Mobilfunkverbindung, die jüngst veröffentlichten Versionen der Übertragungsprotokollstandards nach IEEE 802.11 wie beispielsweise die vom April 2015 sowie eine Kombination aus diesen.

## Revendications

1. Dispositif implantable pour la gestion du contrôle de la vessie, le dispositif implantable comprenant :
un corps ayant une extrémité proximale et une extrémité distale et configuré pour être implanté dans la vessie d'un patient
au moins un capteur de pression (115, 320, 420, 520) positionné le long d'une partie du corps, le capteur de pression (115) étant configuré pour générer une pluralité de signaux de mesure de pression en fonction de la pression appliquée au capteur de pression (115, 320, 420, 520) ;
un processeur (125) monté sur le corps et couplé électriquement à l'au moins un capteur de pression (115, 320, 420, 520), le processeur (125) étant configuré pour transmettre sans fil les signaux de mesure de pression à un dispositif informatique externe (240, 535) pour une sortie ultérieure d'un niveau d'urine dans la vessie sur la base d'un ou de plusieurs des signaux de mesure de pression ;
un élément de retenue positionné le long d'une partie du corps, l'élément de retenue étant configuré pour passer d'une configuration déployée pour retenir le dispositif implantable dans la vessie à une configuration repliée pour permettre au dispositif implantable d'être reçu à l'intérieur et de passer à travers le col vésical et l'urètre, et dans lequel l'extrémité proximale du dispositif implantable comprend un mécanisme de fixation configuré pour s'engager dans un mécanisme de fixation correspondant sur un cathéter de positionnement pour positionner et récupérer le dispositif implantable ;
dans lequel une partie du corps comprend une lumière (310, 410, 525) s'étendant vers l'extrémité proximale et est configurée pour permettre au fluide de s'écouler à travers celle-ci, la lumière (310, 410, 525) est en communication fluidique avec une ouverture définie le long d'une partie du corps ;
un élément de soupape (120, 210, 530, 605, 630, 755, 805) en communication fluidique avec la lumière (310, 410, 525) et couplé électriquement au processeur (125) ; et
dans lequel, à la réception d'une entrée en provenance du dispositif informatique externe (240, 535), le processeur (125) est configuré pour commander le fonctionnement de la soupape (120, 210, 530, 605, 630, 755, 805) pour qu'elle passe d'une position ouverte à une position fermée, et à un nombre quelconque de positions entre celles-ci, pour commander ainsi l'écoulement de fluide à travers la lumière (310, 410, 525).

2. Dispositif implantable selon la revendication 1, comprenant en outre une source d'alimentation (130) couplée au processeur (125) et configurée pour fournir de l'alimentation au moins au capteur de pression (115, 320, 420, 520).

3. Dispositif implantable selon la revendication 2, dans lequel la source d'alimentation (130) comprend une pile.

4. Dispositif implantable selon la revendication 3, dans lequel la pile est rechargeable, de préférence dans lequel le processeur (125) est configuré pour communiquer avec un système de recharge externe et recevoir sans fil de l'énergie de celui-ci pour charger ainsi la pile.

5. Dispositif implantable selon la revendication 4, dans lequel le processeur (125) comprend des composants de recharge inductive.

6. Dispositif implantable selon la revendication 2, comprenant en outre un ou plusieurs fils s'étendant depuis le corps et couplés électriquement au processeur (125) et à la source d'alimentation (130).

7. Dispositif implantable selon la revendication 6, dans lequel, à la réception d'une entrée provenant du dispositif informatique externe (240, 535), le processeur (125) est configuré pour transmettre un signal électrique de la source d'alimentation (130) à l'un ou plusieurs fils, dans lequel au moins un des fils est configuré pour entrer en contact avec une partie interne de la vessie et transmettre le signal électrique à celle-ci pour provoquer une stimulation électrique et une contraction de la vessie.

8. Dispositif implantable selon la revendication 6, dans lequel au moins un des fils comprend une extrémité distale configurée pour s'engager dans une partie interne de la vessie afin de sécuriser le positionnement du dispositif implantable dans la vessie, de préférence dans lequel l'extrémité distale comprend un élément de crochet configuré pour se fixer à une paroi interne de la vessie.

9. Dispositif implantable selon la revendication 1, dans lequel l'extrémité proximale du dispositif implantable comprend un mécanisme de fixation configuré pour s'engager dans un mécanisme de fixation correspondant sur un cathéter de positionnement pour positionner et récupérer le dispositif implantable et dans lequel :
(i) à l'engagement entre les mécanismes de fixation, l'élément de retenue est configuré pour passer à la configuration repliée ; ou
(ii) au désengagement des mécanismes de fixation les uns des autres, l'élément de retenue est configuré pour passer à la configuration déployée.

10. Dispositif implantable selon la revendication 1, dans lequel :
(i) au moins le corps du dispositif implantable est constitué d'un matériau de qualité médicale ;
ou
(ii) le corps du dispositif implantable a une forme ou une géométrie propice à être délivré dans la vessie via un dispositif endoscopique.

11. Dispositif implantable selon la revendication 1, dans lequel le processeur (125) est configuré pour transmettre sans fil des données via un protocole de transmission sans fil choisi dans le groupe constitué par : la communication Bluetooth, la communication infrarouge, la communication en champ proche (NFC), la communication par identification par radiofréquence (RFID), la communication par réseau cellulaire, les versions les plus récemment publiées des normes de protocole de transmission IEEE 802.11 de l'avril 2015, et une combinaison de celles-ci.
